# EUROPEAN PATENT SPECIFICATION

(11) **EP 3 434 169 B1**
(45) Date of publication and mention of the grant of the patent: **19.10.2022**
(21) Application number: 18185923.2
(22) Date of filing: 26.07.2018
(51) Int. Cl.: A61B 1/005, A61M 25/01

(54) **MECHANICAL SYSTEM FOR DISTAL TIP OF A MEDICAL INSERTION TUBE CONTROLLING, EXPECIALLY AN ENDOSCOPE INSERTION TUBE, AND AN ENDOSCOPE HANDLE**
MECHANISCHES SYSTEM FÜR DISTALE SPITZE ZUR STEUERUNG EINES MEDIZINISCHEN EINFÜHRROHRS, INSBESONDERE EIN ENDOSKOPEINFÜHRROHR, UND EIN ENDOSKOPGRIFF
SYSTÈME MÉCANIQUE POUR POINTE DISTALE D'UN TUBE D'INSERTION MÉDICAL COMMANDANT, EN PARTICULIER, UN TUBE D'INSERTION D'ENDOSCOPE ET POIGNÉE D'ENDOSCOPE

(30) Priority: 29.07.2017 PL 42239717
(43) Date of publication of application: 30.01.2019
(73) Proprietor: EndoScope sp. z o.o., 87-100 Torun (PL)
(72) Inventor: Mankowski, Szymon, 87-100 Torun (PL); Mankowski, Patrycjusz, 87-500 Rypin (PL)
(74) Representative: Pawlowska, Justyna

(56) References cited:
- EP-A1- 1 886 617
- EP-A1- 2 745 765
- WO-A1-2015/088142
- US-A1- 2004 059 191
- US-A1- 2014 251 042

## Description

The subject of the invention is a system for controlling the movement of a flexible ending - distal tip of a medical insertion tube, especially an endoscope insertion tube and also an endoscope handle comprising this system for controlling the movement of the flexible end of medical insertion tube. The invention is to be used for medical devices equipped with a insertion tube for diagnostics and medical procedures, in particular for observation and operating on internal organs through natural body cavities. The medical devices are equipped with a movable end where the movement is performed and determined by tension cables - strands.

There are known medical devices equipped with a insertion tube, including endoscopes for examinations and treatments through natural body cavities such as gastroscopes, colonoscopes, duodenoscopes used in gastroenterology; bronchoscopes used in pulmonology or cystoscopes and ureteroscopes used in urology. These devices are equipped with a medical insertion tube, through which channels for media, electrical charge, light, tension cables and surgical instruments are led. Medical insertion tubes for these devices are equipped with a movable end. Moving the tip of the insertion tube is carried out through tension cables led along the entire length of the insertion tube and controlled by knobs located in the handle. The tension cables are placed inside the insertion tube and coupled with the knobs which are controlled and rotated by the user. Each knob is connected with the one end of appropriate tension cable and the other one end to the tip of the insertion tube. The first of the knobs causes the movement of the tip of the endoscope in one of the two planes, referred to as the vertical plane, i.e. up-down, the second knob move the tip in the plane perpendicular to the first defined as the horizontal plane i.e. left-right. Controlling by means of knobs is achieved by pulling and dropping the appropriate tension cables.

Low comfort for the user and insufficient precision is an effect of moving the tip of insertion tube by means of separate knobs controlling the movement of the tension cables. The most problems during medical examination or surgery makes controlling the tip of the insertion tube. During the medical procedure, in case when the tip of insertion tube is deflected by rotation of knobs through tension cables, the user must holds with one hand a handle and with the other hand controls and stabilizes the insertion tube position in a body cavity. User using a hand holding the handle is able to control one knob only, which means he can only move the tip of the insertion tube in one plane. To deflect the tip of the insertion tube in other plane the user must use the other hand.

Endoscopy requires media and tools assistance which are led through the insertion tube to the very end of the device. According to known solutions, controlling media and surgical tools are performed using fingers of both user's hands. The knobs are equipped with interlocks that allow to temporally block of one or both knobs, which slightly improves the operation of the mechanical system but does not eliminate the need to engage both hands. It is not possible to perform the above activities (operate both knobs) simultaneously. The user must periodically release the insertion tube from his hand to use tools or media, or to change the position of the insertion tube tip using both knobs. This reduces the control of the insertion tube movement in vivo, extends the time of examination or surgery, or may even influences of unsuccessful surgery or diagnostics.

In order to carry out an effective, comfortable and first of all precise examination or surgical procedure using medical devices equipped with a movable insertion tube, in particular endoscopes, one-handed operation of the insertion tube must be provided that set goal of the invention.

US8048024 describes a manual control mechanism for medical devices used in vivo, including catheters and endoscopes. The mechanism comprises a housing, a first actuator and a second actuator, the actuators being mutually connected along one plane so that the first actuator moves along a semicircle along the first axis and the second actuator moves along a second axis perpendicular to the first axis. The range of motion of the second actuator is limited by cut out space in the first actuator. The movement of the actuator causes the movement of the moving medical device element and the motion of the first actuator moving the cam with the appropriate pair of tension cables attached to it, causing the movement of the device in the vertical plane, and the motion of the second actuator causes the movement of the device in a horizontal plane by means of direct-attached tension cables to the actuator and led from opposite directions through a pulley. The disadvantage of the device is limiting the movement of one actuator by the other. The range of the actuator movement is the range of shortening the tension cable in a given direction. The dependence of each actuator on another during the movement of the tip of insertion tube in one of the planes can cause unintentional pulling of the tension cables in the second plane and the reduction of the deflection precision of above mentioned tip.

Patent application US20040059191 describes a moving the tip of medical devices by mechanism comprising a rod, which is a movable lever in two planes, i.e. in a vertical plane: up-down and a horizontal plane: left-right, which is fixed by a universal joint to a rod and the rod is terminated with a first pulley . The rod is placed in the slot of the arched arm, which ends with a bevel gear driving the second pulley. Tension cables are wound up on pulleys to move the tip in the vertical and horizontal planes. Up-down movement of the rod causes rotation of the rod with attached pulley to it and wound up up-down tension cable, and the simultaneous movement of the rod in the slot of arched arm does not cause a movement of this arm. The movement of the left-right lever pushes the arched arm, which through the bevel gear drives the second pulley and the left-right tension cable connected to it, the shaft and attached to it pulley do not move. The anatomically conditioned mobility of the fingers limits the possibility of pivoting the lever in that range to of precise motion of the insertion tube tip, which results in ineffective manipulation of the insertion tube tip.

Document WO2015/088142 discloses a joy stick controller for a deflectable tip of an insertion portion. The various axis of the gears lie in one plane.

The invention is defined in claim 1.

Preferably, the driving gear is larger in diameter than the driven gear or the driving gear has a smaller diameter than the diameter of the driven gear.

Preferably, the upper arch guide and the lower arch guide are U-shaped.

Preferably, one tension cable is wound up on each pulley - each pulley is threated with one tie or strand, so that the two ends of the tension cable that are attached to - connected to the distal tip of the insertion tube trigger the tip of the insertion tube in one plane.

Preferably, the upper pins and the lower pins are embedded in - mounted with the base in such a way that in the joint base seats are made and their shape fits - corresponds with the shape of the pins endings allowing for embedding - mounting, and rotating about its own axis.

Preferably, the upper pins and the lower pins are fixed in the base - immovably mounted within the base. The upper arch guide and the lower arch guide is directly and immovably fixed to - connected to at least one driving gear which is mounted movably on the upper pin.

Preferably, four upper pins and four lower pins are mounted within - in or on the base.

Preferably, the upper arch guide is guided movably through the lever above - over the lower arch guide which is guided through the lever via holes - through-holes made within the lever, which are encapsulated with an upper passage and a lower passage, which are movably connected to each other. In the embodiment of the invention the connection is performed by a rotating joint.

Preferably, the upper ach guide is guided movably through the lever above a lower arch guide movably guided through the lever in such a manner that in both arch guides throughout longitudinal through-holes - slots. The slots are created within the guides and through the slots the lever is being moved.

Preferably, a ball joint with three degrees of freedom or a universal joint with two degrees of freedom is used as the joint.

Preferably, a driving gear is mounted on each of the upper pins, and each driving gear forms the gear transmission - gearing - with a driven gear that is mounted to each of the lower pin. Each driven gear is connected with the lower pulley mounted on the pin.

Preferably, on the every single pulley is wound up at least partially one tension cable - means strand or tie, where one end of tension cable which is attached to the tip of the insertion tube causing movement of the tip in one plane.

The endoscope handle connected to the medical insertion tube comprises the mechanical system for controlling of the insertion tube distal tip. The controlling refers to movement of the insertion tube ending by the tension cable - ties or strand connected to the distal tip of the insertion tube. The mechanical system is equipped with a lever movably mounted to a joint with at least two degrees of freedom that is mounted within the base or within - in or on a flexible element - elastic mean - fixed in - mounted within the base. Around the base, at every 90 ° angle, four upper pins are placed and at least two lower pins are placed at a 90 ° angle - are attached to the base at a 90 degrees angle to each other. The upper pins and lower pins are embedded - mounted within the housing and/or embedded within the base. The ends of the upper arch guide are fixed to pair of oppositely located to each other upper pins and two ends of the lower arch guide are fixed to the other pair of oppositely located to each other upper pins. The upper arch guide is led movably through the lever over the lower arch guide which is led through the lever as well. The movement of one of the arch guides or both arch guides simultaneously is caused by/determined by the movement of the lever, and in case when the lever is moved in parallel to the axis of rotation of one of the guide - e.g. first arch guide and perpendicularly to the axis of rotation of other e.g. the second arch guide, only the latter e.g. the second arch guide is being moved. On at least two perpendicular upper pins the driving gears are mounted, wherein every each driving gear forms the gear transmission - gearing - with every each driven gear mounted on lower pin. Each driven gear is connected to the pulley mounted on the lower pin and on that pulley the tension cable is at least partially wound up. The tension cable/tie/strand is at least partially wound around the pulley.

Preferably, the endoscopic control mechanical system for the distal tip of the insertion tube controlling is mounted in the housing in such a way that the upper pins and lower pins of the mechanical system with their outer end are mounted in sockets - holes created in the housing.

Preferably, at the base of the endoscopic mechanical system for controlling the tip of the insertion tube, at every 90° angle four upper pins are mounted and at angle 90° at least two lower pins are mounted - four pins are attached to the base at a 90 degrees angle to each other. The base is connected to at least one wall of the housing.

Embodiments of the invention enable control of the tip of an insertion tube by means of tension cables in medical devices, especially in endoscopes, which are equipped with a movable ending. The invention provides easy to control maneuvering the tip of insertion tube in the full range of motion in a convenient manner, using only one finger. Such a mechanical system provides smooth movement of the tip of insertion tube, which can be pivoted in the range from -180 ° to 180 ° in each plane, according to the direction and level of tilting of the lever constituting the control rod, and thus effective operation of the tip of insertion tube of medical devices. The one-finger control eliminates the need for blocking tension cables, because during a medical procedure, the control rod requires only the finger of one operator's hand to keep constant deflection of the tip of insertion tube. The use of a gearing, especially a multiplying one, which is formed by a larger in diameter gear wheel and a smaller in diameter gear wheel, facilitates the movement of the medical tip of insertion tube, because then small movement of the finger can cover a full range of deflections of tip of insertion tube.

The movement of the tension cables is caused by the deflection of the upper and lower arch guides, and the deflection of arch guides is caused by the movement of the lever. The deflection of each arch guide transfers the movement to the driving gear and the driving gear to the driven gear and by driven gear to pulley. Each of arch guides is independent from other, whereby the movement of the lever in the planes containing the axis of rotation of the upper arch guide causes a movement of second arch guide so lower arch guide is tilted but the upper arch guide stays still. Analogically, by moving the lever in the planes containing the axis of rotation of lower arch guide, the upper arch guide is tilted and the lower one remains stationary. In cases where no axis of rotation of any of the arch guides is located on the lever movement planes, both arch guides are tilted simultaneously but independently of each other.

The arch guide may transfer the movement to the driven gear by the rotation of the movable upper pins on which is mounted together with the driven gear or in such a way that the end of the arch guide is connected to the driving gear and rotate together around the stationary fixed upper pins. The driving gear cause a motion of the driven gear. Rotation of driven gear can be transferred to the pulleys by rotating the lower pin or directly by attaching the driven gear to the pulley so then can both rotating together about the stationary lower pin.

Connections between upper pins and arch guides, upper pins and driving gears, upper arch guide and driving gears, lower pins and driven gears, lower pins and pulleys, pulleys and driven gears, base and pins, as well as pins with housing, can be implemented in many known ways so that, finally, the movement of the lever through movable arch guides can be transferred to the movement of the driving gear coupled to the driven gear which is connected to the pulley. The rotation of the pulley causes pulling the tension cables. Among the potentially useful types of connections can be applied kinematic or static connections such as inlet, spline and pin type, as well as non-separable, including glued, welded connections. Connections of the above mentioned elements of the control system have o provide the rotation of the appropriate pulley with wound up tension cable and deflecting the tip of insertion tube in the direction given by the lever.

Embodiments of the invention are presented with more details in the drawings where:
Fig. 1 shows an axonometric view of the mechanical system according to Example 1.
Fig. 2 axonometric view of the lever in the form of a control rod.
Fig. 3 a cross-section through the mechanical system according to Example 1 and its housing.
Fig. 4 is an axonometric view of a medical device equipped with a insertion tube with movable end.
Fig. 5 an axonometric view of the mechanical system according to Example 1 with lever position towards the bottom and axonometric view of the tip of insertion tube in a downward bend in.
Fig. 6 axonometric view of the mechanical system according to example 1 with the lever position in the right direction and the tip of insertion tube bend in right direction.
Fig.7 an axonometric view of the mechanical system according to example 1 with the lever in position between the right and top direction and the tip of insertion tube deflected in between the top and right direction.
Fig. 8 and Fig. 9 an axonometric view of the mechanical system according to Example 2.
Fig. 10 an axonometric view of the mechanical system according to example 2 with the lever position towards the bottom and the tip of insertion tube bended down.
Fig. 11 axonometric view of the mechanical system according to example 2 with the lever position in the right direction and the tip of insertion tube in the right bend.
Fig.12 axonometric view of the mechanical system according to example 2 with lever position in the direction between left and down direction and the tip of insertion tube bended between left and bottom directions.
Fig. 13 an axonometric view of the mechanical system according to example 3.
Fig. 14 an axonometric view of the mechanical system according to an example 4.
Fig. 15 an axonometric view of the mechanical system according to Example 5.
Fig. 16, a cross-section through the mechanical system with housing according to example 6.
Fig. 17 a cross-section through the mechanical system and its housing according to example 7.

### Example 1

### a) Detailed description of design of mechanical system for controlling the tip of insertion tube.

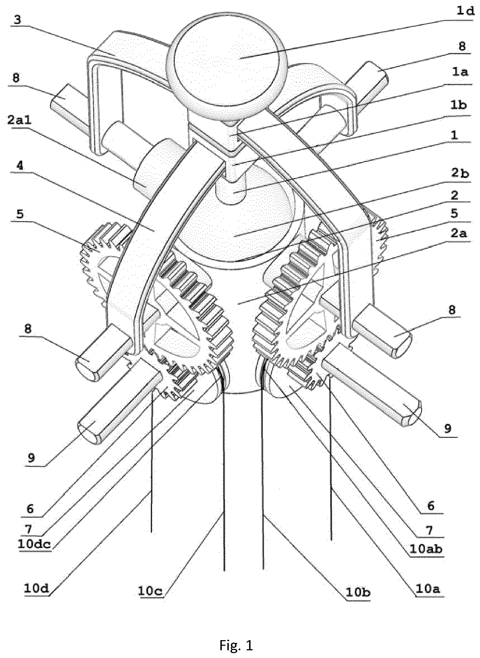

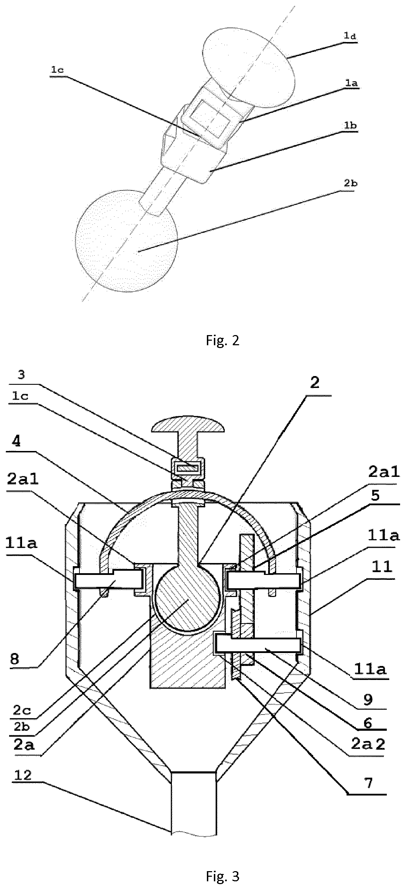

As shown in Fig. 1, the device for controlling the movement of the tip of medical insertion tube constituting the medical insertion tube control mechanical system is equipped with a lever 1 which is a cylindrical control rod terminated on one end with a knob 1d providing support for the operator's finger and on the other hand embedded in the base 2a on the a ball joint 2 which has three degrees of freedom, which is formed by a hemispherical bed 2c and a spherical head 2b mounted therein. The hemispherical bed 2c is formed in the base 2a of the mechanical system as shown in fig. 3.

In the upper part of the lever 1 through-holes are provided, housed by a upper passage 1a and a lower passage 1b, through which the upper arch guide 3 and the lower arch guide 4 are guided, in such a way that the axes of rotation of both arch guides are perpendicular to each other. Both arch guides have a U-shaped form, while on the section cooperating with the lever 1 they have a shape of part of a ring. As shown in fig. 2, the upper passage 1a and the lower passage 1b in the lever 1 are connected movably via rotating joint 1c, which allows them to rotate about common axis of rotation.

Sockets with circular holes are formed in the base 2a of the mechanical system: four upper sockets 2a1 arranged at an angle of 90° and two lower sockets 2a2 arranged at 90°, in which inner ends of four upper pins 8 and two lower pins 9 are inserted. The sockets have an internal form adapted to the ends of the pins to guarantee free rotation of the pins embedded in these sockets. The upper pins 8 and the lower pins 9 can rotate freely in the sockets around their own axes of rotation.

The upper pins 8 and the lower pins 9 are also embedded with their outer ends in blind holes 11a made in the housing 11 of the entire mechanical system, as shown in Fig. 3. The upper pins 8 and the lower pins 9 with their outer ends can rotate freely in housing holes 11a around their own rotation axis.

The upper arch guide 3 is guided through the upper passage 1a, and its ends are fixed by a static connection on two coaxially placed upper pins 8. The lower arch guide 4 is led through the lower passage 1b and its ends are fixed by a static connection on another two upper pins 8 coaxially placed as well.

On the two upper pins 8 perpendicular to each other are fixed by static connection, driving gears 5, which form gear transmissions with corresponding driven gears 6 also supported by static connection on the lower pins 9 so that the gears move with the movement of the pins. Driving gear 5 with a larger diameter than the diameter of the driven gear 6 coupled to it forms a multiplying transmissions. Driving gear 5 with smaller diameter than that connected to it driven gear 6 can be applied and then forms a reducing transmission.

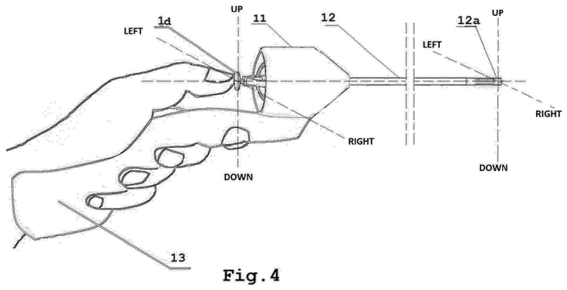

The pulley 7 is fixed to each driven gear 6, which is mounted on the lower pin 9 in such a way that each driven gear 6 is coupled to the pulley 7 and moves with the rotation of the lower pin 9. As shown in Fig. 1 and 4, one tension cable is wound up in a halfway on each pulley 7 in such a manner that each tension cable on the pulley have two free ends, which are then guided through the insertion tube 12 to the movable end 12a e.g. the flexible end and attached they are to its distal tip. On one of the pulleys 7 one up-down tension cable 10ab is wound up with two ends whose, when they are pulled or released, cause movement of the movable end of insertion tube in one plane, i.e. the up end 10a and the down end 10b allow the tip of the medical insertion tube to move in a set direction in the vertical plane up-down. On the other pulley 7 similarly one right-left tension cable 10cd is wound up with two ends: the right end 10c and the left 10d end, which allow the tip of the medical insertion tube to move in a set direction in the horizontal plane, i.e. left-right.

Mechanical system is enclosed in a housing 11 to forming a handle provided with a insertion tube. The mechanical system is designed for controlling medical devices such as, for example, endoscopes equipped with a insertion tube 12 with a movable end 12a, which can be also a flexible end as shown in Fig. 4. The housing 11 of the handle 13 is connected to the proximal end of the insertion tube 12. The cables are led inside insertion tube 12 to the movable end 12a and fixed at its distal tip.

### b) Principles of operation of the mechanical system

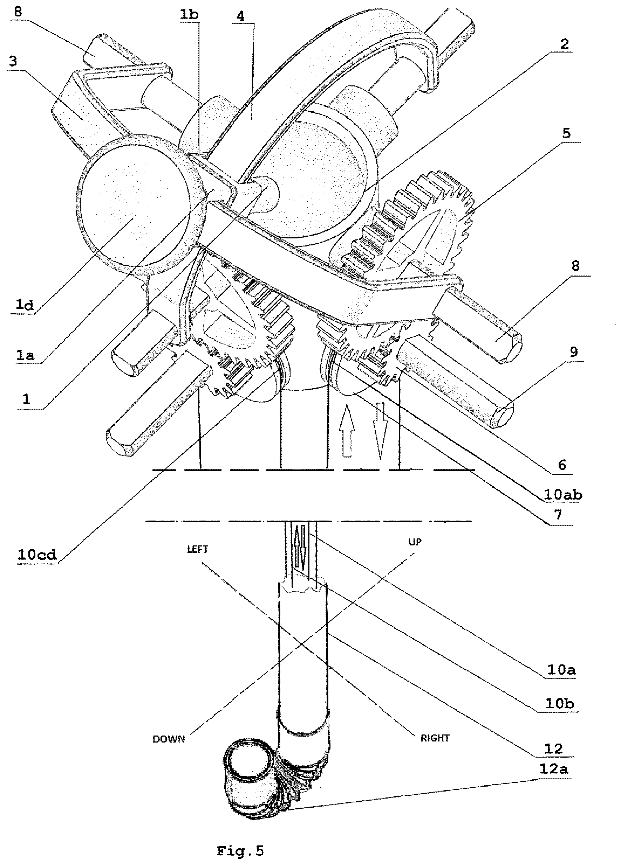

By moving the 1d knob, the operator moves the lever 1 constituting the control rod in the given direction. The lever 1, thanks to that is settled on the ball joint 2, can tilt in all directions. As shown in Fig. 5, 6, 7, the movement of the lever 1 in a predetermined direction causes the upper arch guide 3 or the lower arch guide 4 or both arch guides to be deflected at the same time, transferring the movement of the lever 1 into the move of the upper pins 8 and the lower pins 9.

As it is shown in Fig. 4, moving the lever 1 in the predetermined direction finally causes the movement of the tip of the insertion tube. In the case of an endoscope is equipped with a movable end 12a of the insertion tube 12, the control mechanical system causes the movable end 12a to be bent in all directions analogously to the movement of the lever. The movable end of the insertion rube can be deflected in a designated direction in the range of -180 ° to 180 ° in each plane.

As shown in Fig. 5, the movement of the lever 1 in the vertical plane having the axis of rotation of the lower arch guide 4, downwards from the operator side of the lever 1 and perpendicular to the axis of rotation of the upper arch guide 3, causes the upper arch guide 3 to move downwards - the upper passage 1a is positioned along the axis of rotation of the upper arch guide 3 and perpendicular to the axis of rotation of the lower arch guide 4. Movement of the lever 1 downwards in the plane having the axis of rotation of the lower arch guide 4 does not cause a movement of the lower arch guide. The described independency of movements of the arch guides is carried out by sliding the lower passage 1b along the lower arch guide 4 - lower passage 1b is aligned along the axis of rotation of the lower arch guide 4 and perpendicular to the axis of rotation of the upper arch guide 3. Movement of the upper arch guide 3 causes the rotation of two coaxially placed upper pins. The driving gear 5 transmits rotation to the driven gear 6, which causes the rotation of one lower pin 9 and at the same time the pulley 7 connected to the driven gear 6. The pulley 7 rotating causes movement of the tension cable up-down 10ab making one end shorter the down-end 10b and extending other end, the up-end 10a which causes deflection of the movable end 12a of the insertion tube 12 in the vertical plane in the down direction. The lower arch guide 4 and the left-right 10cd tension cable are not moving.

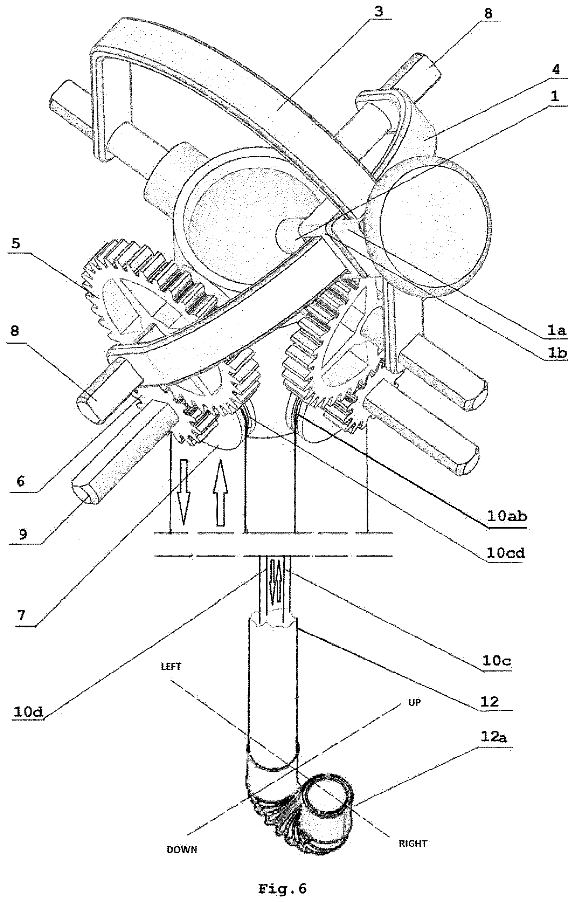

As shown in Fig. 6, the deflection of the lever 1 in the horizontal plane in the right direction finally results in the corresponding deflection of the movable end 12a of the insertion tube 12 in the horizontal plane in the right direction. The lever 1 during movement causes the lower arch guide 4 to move in the right direction - the lower passage 1b is set along the axis of rotation of the lower arch guide 4 and perpendicular to the axis of rotation of the upper arch guide 3. Movement of the lever 1 in the right direction does not cause movement of the upper arch guide 3, which is allowed by sliding the upper passage 1a along the upper arch guide 3 - the upper passage 1a is positioned along the axis of rotation of the upper arch guide 3 and perpendicular to the axis of rotation of the lower arch guide 4. Movement of the lower arch guide 4 causes the rotation of the two coaxially placed pins 8 on which arch guide 4 is seated actuate the driving gear 5 which is mounted on one of those pins. The driving gear 5 transmits the rotation to the driven gear 6, which causes also the rotation of one lower pin 9 and the pulley 7 at the same time. The pulley 7 through rotation moves wound up tension cable left-right 10cd making shorter the right-end 10c, while extending the left-end 10d which bends the movable end 12a of the insertion tube 12 in the horizontal plane in the right direction. The upper arch guide 3 and the up-down 10ab are not moving.

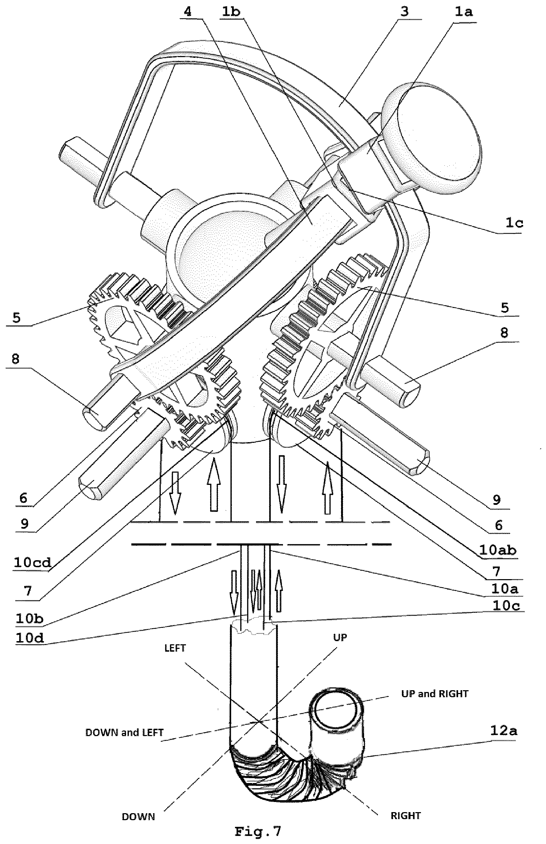

There is possibility to tilt the lever 1 in the intermediate planes, i.e. not including the axis of rotation of the upper arch guide 3 or the lower arch guide 4, which causes simultaneous movement of both of these guides. The effect of the simultaneous movement of both guides is to deflecting the movable end 12a of the insertion tube 12 in the intermediate planes between the vertical and horizontal planes, in the direction analogous to the tilt direction of the lever 1. As shown in Fig. 7, the lever 1 is tilted in the plane not containing the axis of rotation of any guides e.g. in the direction defined between the right and the up direction, causes finally deflection of the movable end 12a of the insertion tube 12 in the direction determined between the right and the up direction. The lever 1 moving in direction right-up drives the lower arch guide 4 in the right direction and simultaneously moves the upper arch guide 3 towards direction up. Simultaneous movement of the upper arch guide 3 and lower arch guide 4 causes a change of the angle between their sections cooperating with the lever 1. The change of this angle is possible by the upper passages 1a and the lower passage 1b of the lever 1, which slide on arch guides and rotate on the rotating joint placed between them 1c. The angle of their mutual rotation corresponds to the change in the angle between the upper and lower arch guides 4. The simultaneous movement of both upper arch guides 3 and lower arch guides 4 causes rotation of the four upper pins 8 together with two driving gears 5 mounted on two of them and placed every 90 degrees. The two driving gears 5 transmit the rotary motion to the two driven gears 6, which causes the rotation of the two lower pins 9 and simultaneously the rotation of the two pulleys 7. The pulleys 7 through rotation drive wound up on them tension cables. The pulley 7 with the wound up up-down tension cable 10ab by rotation pull the end of the up-end 10a and release the down-end 10b. The second pulley 7 with the right-left wound up tension cable 10cd rotating pull the right-end 10c and release the left-end 10d. Simultaneous pulling of the end of the up-end 10a and the right-end 10c causes the movable end 12a of the insertion tube 12 to deflect between the up and the right direction in the intermediate plane between the vertical and horizontal planes.

### Example 1

### a) Detailed description of design of mechanical system for controlling the tip of insertion tube.

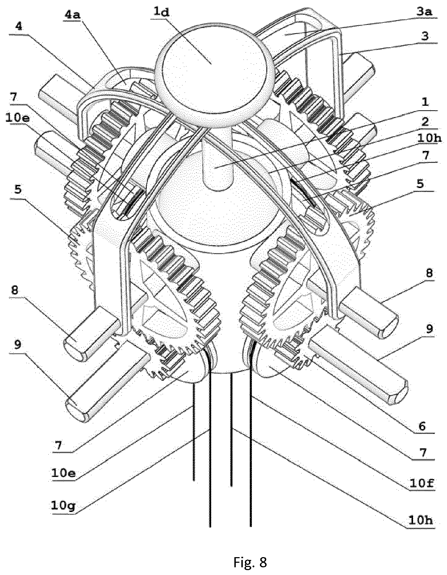

As shown in Fig. 8, the mechanical system for controlling the movement of the tip of the medical insertion tube in another embodiment is equipped with a lever 1 which is a cylindrical control rod terminated on one end with a knob 1d supporting the operator's finger and on the other hand embedded in the base 2a on the ball joint 2 with three degrees of freedom, which is formed by a hemispherical bed 2c and a spherical head head 2b embedded therein. The hemispherical bed 2c is formed in the base 2a of the mechanical system, as shown in Fig. 3.

A upper arch guide 3 and a lower arch guide 4 are guided through the upper part of the lever 1, each of which is mounted on oppositely located to each other upper pins 8. In the upper arch guide 3 and the lower arch guide 4 through-slots 3a and 4a are formed through which the lever 1 is guided. These slots allow to move the lever 1 in all directions and consequently pushing only one or the other or both arch guides at the same time. Both arch guides have a U-shaped form, while on the section cooperating with the lever 1 they have a shape of a part of a ring.

In the base 2a of the mechanical system four upper sockets 2a1 and four lower sockets 2a2 are formed every each with circular blind hole and to these sockets by kinematic connection mounted with their inner ends four upper pins 8 and four lower pins respectively 9. The shape of the sockets is adapted to the shape of the ends of pins allowing their rotation in sockets. The upper pins and the corresponding sockets are placed at every 90°. The lower pins 9 and the corresponding sockets are placed at angle 90°. The upper pins 8 and the lower pins 9 by their outer ends are mounted by a kinematic connection in the circular blind holes 11a made in the housing 11 of the mechanical system. The deposition of the pins in the housing is analogous to the first example, as shown in Fig. 3.

The ends of the upper arch guide 3 are fixed by a static connection on two oppositely located to each other upper pins 8. The lower arch guide ends 4 are fixed by a static connection on two another oppositely located to each other upper pins 8. On each upper pin 8 driving gear 5 is mounted by a static connection. Each the driving gear 5 forms gear transmissions with respective driven gear 6 mounted by a static connection on the lower pins 9, so that the gears move with the movement of the pins. A driving gear 5 of larger diameter coupled to the driven gear 6 which has smaller diameter form a multiplying gear transmissions. In another embodiment, driving gear 5 of smaller diameter coupled to the driven gear 6 which has larger diameter form a reduction gear transmissions. Each driven gear 6 is coupled to the pulley 7 and fixed by a static connection on the common lower pin 9 and rotates with this pin.

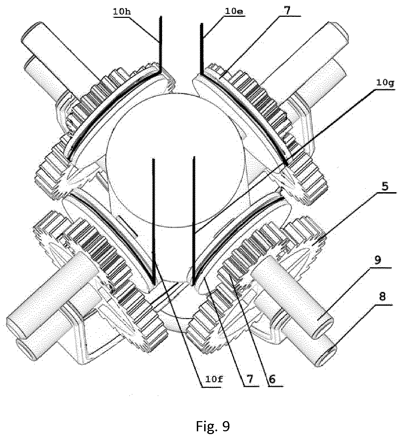

On each of the four pulleys 7 one of the four tension cables corresponding to the direction of movement of the movable end 12a is fixed: upper tension cable 10e, lower tension cable 10f, right tension cable 10g, left tension cable 10h. The tension cables are only partially wound up onto the pulleys in such a way that one end of each tension cable is permanently fixed on the pulley and the other end passes through the insertion tube 12 to the movable end 12a of the insertion tube 12 and is attached to its distal end. As shown in Fig. 9, the upper tension cable 10e and the lower tension cable 10f deflect the movable end in a predetermined direction in the vertical plane and are partially and counterdriven wound up on the pulleys 7 mounted on coaxially placed lower pins 9. The right tension cable 10g and the left tension cable 10h deflect the movable end in the horizontal plane and are partially and counterdriven wound up on the next two pulleys 7 mounted on two further coaxially placed lower pins 9.

Mounting and using the mechanical system in housing and in medical device equipped with a movable end of insertion tube is analogous to example 1.

### b) Principles of operation of the mechanical system

Control of insertion tube tip is performed analogously to the one described in the first example by moving the lever 1 in any directions in the sockets 3a and 3b of the respective arch guides, causing a movement of the upper arch guide 3 or the lower arch guide 4, or both simultaneously, analogously as described in example 1.

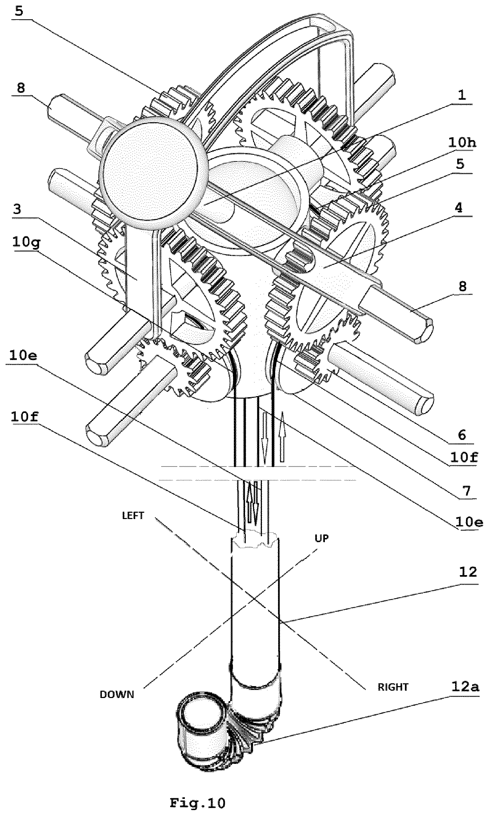

As shown in Fig. 10, the tilting of the lever 1 in the vertical plane containing the axis of rotation of the upper guide 3 in direction down and perpendicular to the axis of rotation of the lower arch guide 4 moves the lower arch guide 4 downwards and, as a result, the rotation of the two driving gears 5 mounted on the same upper pins 8 on which the lower arch guide 4 is mounted. The driving gears 5 moved together with the lower arch guide 4 set in motion driven gears 6 which are coupled to driving gears 5. The movement of the driven gears 6 causes the movement of two opposite pulleys 7 mounted on oppositely located to each other pins 9. As shown in Fig. 9, the movement of the opposing pulleys 7 sets in motion a partially and counterdriven wound up up upper tension cable 10e and lower tension cable 10f.

The first pulley 7 by rotation pull and this way makes shorter lower tension cable 10f, the other rotating at the same time releases of the upper tension cable 10e, which causes deflection of the movable end 12a of the insertion tube 12 in the vertical plane in the direction down. The upper arch guide 3 and the right tension cable 10g and the left tension cable 10h stay still.

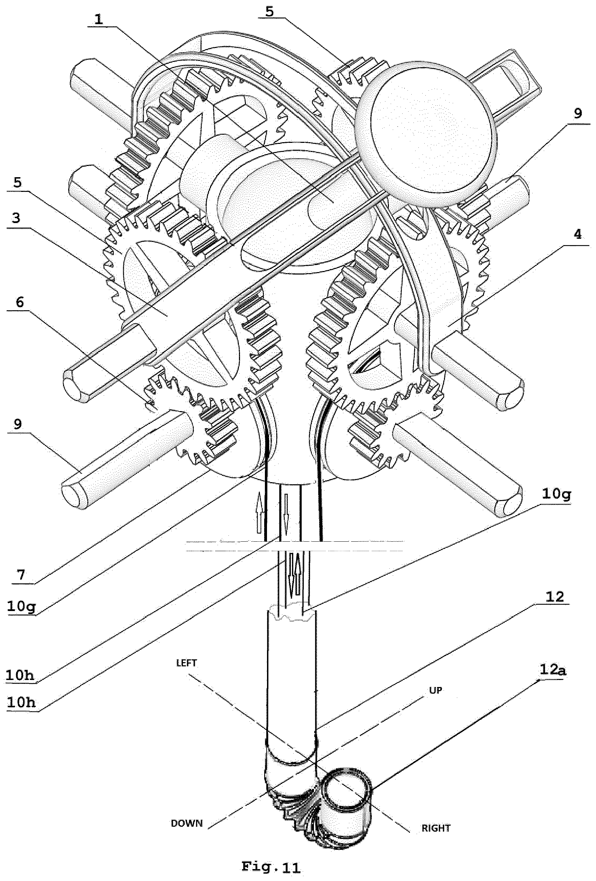

As shown in Fig. 11, the tilting the lever 1 in the horizontal plane containing the axis of rotation of the lower arch guide 4 in the right direction and perpendicular to the axis of rotation of the upper arch guide 3 moves the upper arch guide 3 in the right direction and in effect turns two driving gears 5 mounted on the same upper pins 8, on which the upper arch guide 3 is mounted. The driving gears 5 moved together with the upper arch guide 3 actuate coupled to these the driven gears 6. The movement of the driven gears 6 involves the movement of opposing pulleys 7 mounted on oppositely located to each other lower pins 9. As shown in Fig. 9, the movement of the opposing pulleys 7 sets in motion a partially and counterdriven wound up right tension cable 10g and the left tension cable 10h.

The first rotating pulley 7 by its rotation make shorter the wound up on that pulled right tension cable 10g, while the second pulley 7 rotates at the same time but releases wound up left tension cable 10h, which causes the deflection of movable end 12a of the insertion tube 12 in the horizontal plane in the direction right. Lower arch guide 4 and upper tension cable 10e and lower tension cable 10f stay still.

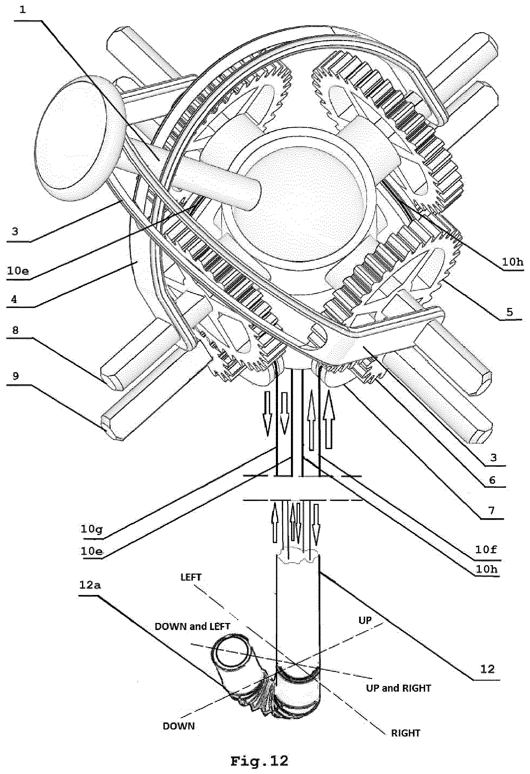

As shown in Fig. 12, the deflection of the lever 1 in the planes without the axis of rotation of the upper arch guide 3 and the lower arch guide 4 in the direction defined between left and down direction causes the upper arch guide 3 to move downwards and the lower arch guide 4 in the left direction what causes the rotation of all four upper pins 8 and, as a result, rotation of all driving gears 5 and all of coupled with them driven gears 6. This causes rotation in four pulleys 7 mounted on lower pins 9. Each of the pulleys 7 rotating makes the length of tension cables shorter or longer in such a way that, moving the upper arch guide 3 downwards causes the lower tension cable 10f to be pulled and a release of upper tension cable 10e what causes deflection of movable end 12a downwards.

Moving the lower arch guide 4 in the left direction makes the left tension cable 10h shorter and the extension of the right tension cable 10g, which causes pulling movable end 12a towards the left.

The effect of simultaneous pulling of the lower tension cable 10f and the left tension cable 10h is deflection of the tip of the movable end 12a of the insertion tube 12 between left and down direction in the intermediate plane between the vertical and horizontal planes.

### Example 3

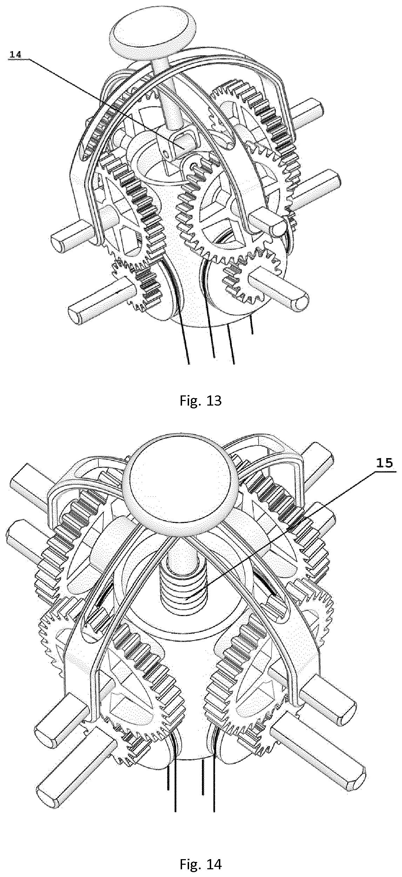

The mechanical system is constructed as described in example 1 or 2 and is shown in fig. 13 but here the lever 1 constituting the control rod is mounted in the base 2a on the universal joint 14 with two degrees of freedom.

The essence of operation is analogous to that described in example 1 or 2.

### Example 4

The mechanical system is constructed as described in example 1 or 2 and is shown in fig. 14, except that the lever 1 constituting the control rod is mounted in the base 2a on the elastic element in the form of a bellows made of a selected elastomer acting like a ball joint and capable of tilting the lever 1 in all directions. The flexible element 15 is mounted on the base 2a of the mechanical system.

The essence of operation is analogous to example 1 or 2.

### Example 5

### a) Detailed description of design of mechanical system for controlling the tip of insertion tube.

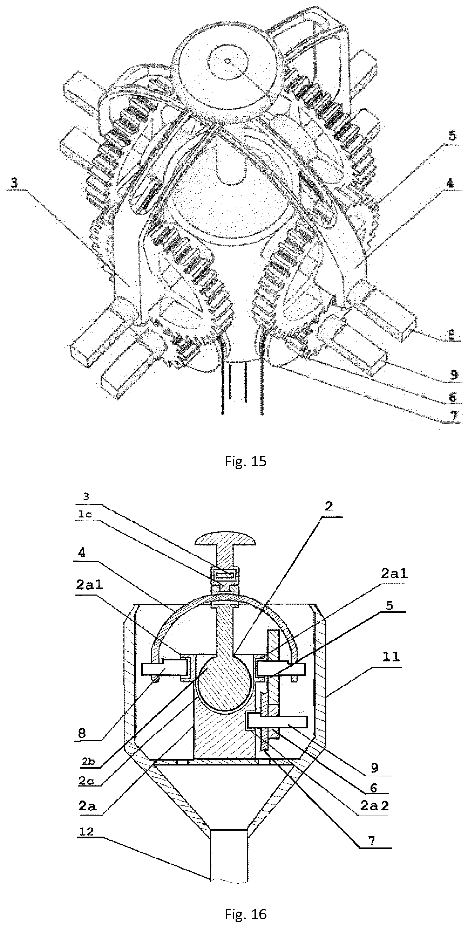

The mechanical system is constructed as described in Examples 1-4 or 2 and is shown in Fig. 15 with the difference that in the base 2a of the mechanical system four upper sockets 2a1 and the four lower sockets 2a2 are made with polygonal in cross-sections blind holes to which with their inner ends, respectively, four upper pins 8 and four lower pins 9. The shape of the holes in sockets is adapted to the shape of the ends of the pins forming a shape-connection that prevents them from rotating in the sockets. The upper pins 8 and the lower pins 9 are also mounted with their outer end by a shape-connection in holes with a polygonal cross-section 11a adapted to the shape of the pins ends and made in the housing 11 of the mechanical system.

The ends of the upper arch guide 3 are mounted by a kinematic connection on two oppositely located to each other upper pins 8 and are connected by a static connection with driving gears 5. The lower arch guide ends 4 are also mounted by a kinematic connection on two another oppositely located to each other upper pins 8 and are also statically connected with driving gears 5. Each driving gear 5 forms gear transmissions with respective driven gear 6 mounted by a kinematic connection on the lower pins 9. Each driven gear 6 is inseparable from the pulley 7 and is mounted by a kinematic connection on a common lower pin 9.

### b) Principles of operation of the mechanical system

The essence of operation is analogous to example 1 or 2 except that the movement of the upper arch guide 3 and the lower arch guide 4 is transferred to the movement of the respective tension cables by rotating the driving gears 5 and the driven gears 6 connected to the pulleys 7 in such a way that they rotate around the fixed upper pins 8 and the lower pins 9.

### Example 6

The mechanical system is constructed as described in the above examples, and the mounting of the system in the handle of the medical device provided with the insertion tube is carried out in such a way that the upper pins 8 and lower pins 9 are mounted only in the base 2a of the mechanical system, and the mounting of the mechanical system in the housing 11 is realized by combining the base 2a of the mechanical system with the housing wall 11, as shown in Fig. 16

### Example 7

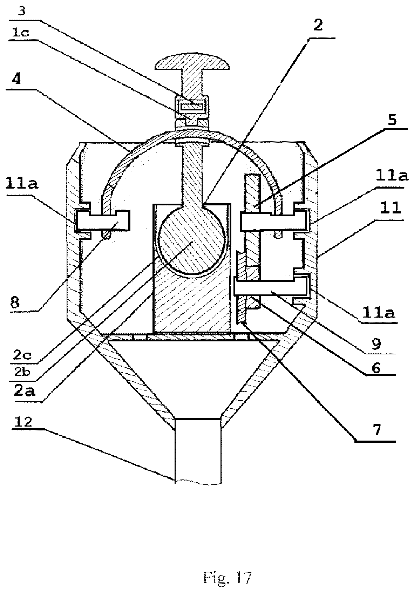

The mechanical system is constructed as described in the above examples, and the mounting of the system in the handle of the medical device provided with the insertion tube is realized in this way, the upper pins 8 and lower pins 9 are mounted only in the housing 11, and the base 2a of the control system is mounted on the wall of the housing 11, as shown in Fig. 17.

## Claims

1. Mechanical system for controlling movement of a distal tip of a medical insertion tube, comprising a movable lever and pulleys that are threaded with tension cables adapted to be connected with the deflectable distal tip of the insertion tube, wherein the lever (1) is movably mounted to a joint of at least two degrees of freedom that is mounted within a base (2a), wherein four upper pins (8) are attached to the proximal part of
the base (2a) at a 90 degrees angle to each other and at least two lower pins (9) are attached to the distal part of
the base (2a) at a 90 degrees angle to each other, and an upper guide (3) endings are attached to two oppositely located to each other upper pins (8) and a lower guide (4) endings are attached to the other two oppositely located to each other upper pins (8), whereas the upper guide (3) is movably guided through the lever (1) and the upper guide (3) is located over the lower guide (4) that is movably guided through the lever (1) and a movement of one of the guides or a simultaneous movement of both guides are determined by a movement of the lever (1) wherein in the situation in which the lever is being moved in parallel to the rotation axis of one of the guides and perpendicularly to the rotation axis of the other guide, only the latter guide is being moved and furthermore driving gears (5) are mounted to at least two perpendicularly located to each other upper pins (8) and each driving gear (5) forms a gearing with a driven gear (6) mounted to the corresponding lower pin (9), and each driven gear (6) is connected with the pulley (7) which is mounted to the lower pin (9) and the tension cable is at least partially wound around the pulley (7) .

2. The system according to the claim 1, wherein a diameter of the driving gear (5) is larger than a diameter of the driven gear (6) or a diameter of the driving gear (5) is smaller than a diameter of the driven gear (6).

3. The system according to the claim 1, wherein the upper guide (3) and the lower guide (4) are U-shaped.

4. The system according to the claim 1, wherein each pulley (7) is threaded with one cable in such a way that two ends of the cable that are connected to the distal tip of the insertion tube provide the movement of the distal tip of the insertion tube in one plane.

5. The system according to the claims 1 or 2 or 3 or 4, wherein the upper pins (8) and the lower pins (9) are mounted within the base (2a) in such a way that sockets are formed within the base (2a) and the shape of the sockets corresponds with the shape of the pins endings for enabling them to be mounted and rotated about their axes.

6. The system according to the claims 1 or 2 or 3 or 4, wherein the upper pins (8) and the lower pins (9) are immovably mounted within the base (2a), wherein each of the guides (3,4) is directly and immovably connected to at least one driving gear (5) that is mounted movably to the upper pin (8).

7. The system according to any of the claims 1-6, wherein four upper pins (8) and four lower pins (9) are mounted within the base (2a).

8. The system according to any of the claims 1-7, wherein the upper guide (3) is guided movably through the lever (1), and the upper guide (3) is located above the lower guide (4) that is movably guided through the lever (1) and the guides (3, 4) are movably guided through the lever (1) with the through-holes of the lever (1) that are enclosed by an upper passage (1a) and a lower passage (1b) that are movably connected.

9. The system according to the any of claims 1-7, wherein the upper guide (3) is guided movably through the lever (1), and the upper guide (3) is located above the lower guide (4) that is movably guided through the lever (1), and the guides (3, 4) are movably guided through the lever (1) in such a way that longitudinal through-slots (3a, 4a) are formed within the upper guide (3) and the lower guide (4) which the lever (1) is being moved within.

10. The system according to the claim 1, wherein a ball joint (2) of three degrees of freedom or a universal joint (14) of two degrees of freedom.

11. The system according to the claim 7, wherein the driving gear (5) is mounted to each of the upper pins (8) and each driving gear (5) forms the gearing with a driven gear (6) that is mounted to each of the lower pins (9), wherein each driven gear (6) is connected with the pulley (7) that is mounted to the lower pin (9).

12. The system according to the claim 11, wherein each pulley (7) is at least partially threaded with one tension cable wherein one of the tension cable ends is fixed to the distal tip of the insertion tube that provides the movement of the tip in one plane.

13. An endoscope handle connected to the medical insertion tube wherein a mechanical system defined in any of claims 1 to 12 is enclosed in a housing (11) of the handle and the upper pins (8) and the lower pins (9) of the mechanical system are combined with the housing (11).

14. The endoscope handle according to the claim 13, wherein the system for the distal tip of the insertion tube controlling is mounted in the housing (11) in such a way that the upper pins (8) and the lower pins (9) of the system are positioned with their outer endings in holes formed in the housing (11).

15. The endoscope handle according to the claim 13, wherein four upper pins (8) are attached to the base (2a) at a 90 degrees angle to each other and at least two lower pins (9) are attached to the base (2a) at a 90 degrees angle to each other and the base (2a) is connected with at least one wall of the housing (11).

## Patentansprüche

1. Mechanisches System zur Steuerung der Bewegung des weiteren Endes einer medizinischen Sonde, das einen beweglichen Hebel und Rollen umfasst, auf denen Zugbänder aufgewickelt sind, die mit dem schwenkbaren weiteren Ende der Sonde verbunden sind, wobei, dass der Hebel (1) beweglich an einem Gelenk mit mindestens zwei Freiheitsgraden angebracht ist, das an einem Ständer (2a) montiert ist,
und an dem näheren Teil des Ständers (2a) vier obere Bolzen (8) in einem Winkel von 90 Grad zueinander und an dem weiteren Teil des Ständers (2a) mindestens zwei untere Bolzen (9) in einem Winkel von 90 Grad zueinander befestigt sind, wobei außerdem die Enden der oberen Führungsschiene (3) an zwei gegenüberliegenden oberen Bolzen (8) und die Enden der unteren Führungsschiene (4) an den anderen beiden gegenüberliegenden oberen Bolzen (8) befestigt sind, wobei die obere Führungsschiene (3) durch den Hebel (1) oberhalb der durch den Hebel (1) beweglich geführten unteren Führungsschiene (4) beweglich geführt ist und außerdem die Bewegung eines oder beider Führungsschienen gleichzeitig durch die Bewegung des Hebels (1) bewirkt wird und außerdem, wenn der Hebel (1) parallel zur Drehachse einer der Führungsschienen und senkrecht zur Drehachse der anderen Führungsschiene bewegt wird, nur die letztere Führungsschiene bewegt wird
und zusätzlich an zwei senkrecht angeordneten oberen Bolzen (8) Antriebszahnräder (5) angebracht sind, und jedes Antriebszahnrad (5) ein Zahnrad mit einer entsprechenden, am entsprechenden unteren Bolzen (9) angebrachten angetriebenes Zahnrad (6) bildet, und jedes angetriebenes Zahnrad (6) mit einer am unteren Bolzen (9) angebrachten Rolle (7) verbunden ist, auf die ein Zugband zumindest teilweise aufgewickelt ist.

2. Anordnung nach Anspruch 1, wobei dass der Durchmesser des Antriebszahnrades (5) größer ist als der Durchmesser des Abtriebszahnrades (6) oder der Durchmesser des Antriebszahnrades (5) kleiner ist als der Durchmesser des angetriebenen Zahnrades (6).

3. Anordnung nach Anspruch 1, wobei dass die obere Führungsschiene (3) und die untere Führungsschiene (4) U-förmig sind.

4. Anordnung nach Anspruch 1, wobei dass auf jede der Rollen (7) ein Zugband so aufgewickelt ist, dass die beiden Enden des Zugbandes, die mit dem weiteren Ende der Sonde verbunden sind, die Bewegung des weiteren Endes der Sonde in einer Ebene gewährleisten.

5. Anordnung nach Anspruch 1 oder 2 oder 3 oder 4, wobei dass die oberen Bolzen (8) und die unteren Bolzen (9) so im Ständer (2a) sitzen, dass im Ständer (2a) Aufnahmen vorhanden sind, deren Form derjenigen der Enden der Bolzen entspricht, um deren Sitz und Drehung um ihre eigene Achse zu ermöglichen.

6. Anordnung nach Anspruch 1 oder 2 oder 3 oder 4, wobei dass die oberen Bolzen (8) und die unteren Bolzen (9) im Ständer (2a) befestigt sind, wobei jede der Führungsschienen (3, 4) direkt und unbeweglich mit mindestens einem Antriebsrad (5) verbunden ist, das beweglich auf dem oberen Bolzen (8) befestigt ist.

7. Anordnung nach einem der Ansprüche 1 bis 6, wobei dass vier obere Bolzen (8) und vier untere Bolzen (9) am Ständer (2a) montiert sind.

8. Anordnung nach einem der Ansprüche 1-7, wobei dass die obere Führungsschiene (3) durch den Hebel (1) beweglich geführt ist und die obere Führungsschiene (3) oberhalb der unteren Führungsschiene (4) liegt, die durch den Hebel (1) beweglich geführt ist, und die Führungsschienen (3, 4) durch den Hebel (1) beweglich geführt werden, wobei die Durchgangslöcher des Hebels (1) in der beweglich verbundenen oberen Durchführung (1a) und unteren Durchführung (1b) liegen.

9. Anordnung nach einem der Ansprüche 1-7, wobei dass die obere Führungsschiene (3) durch den Hebel (1) beweglich geführt ist und die obere Führungsschiene (3) oberhalb der unteren Führungsschiene (4) liegt, die durch den Hebel (1) beweglich geführt ist, und die Führungsschienen (3, 4) durch den Hebel (1) derart beweglich geführt werden, dass in der oberen Führungsschiene (3) und der unteren Führungsschiene (4) Längsdurchgangsschlitze (3a, 4a) gebildet werden, in denen sich der Hebel (1) bewegt.

10. Anordnung nach Anspruch 1, wobei dass ein Kugelgelenk (2) mit drei Freiheitsgraden oder ein Universalgelenk (14) mit zwei Freiheitsgraden verwendet wird.

11. Anordnung nach Anspruch 7, wobei dass das Antriebszahnrad (5) auf jedem oberen Bolzen (8) sitzt und jedes Antriebszahnrad (5) mit einem angetriebenen Zahnrad (6), das auf jedem unteren Bolzen (9) sitzt, ein Getriebe bildet, wobei jedes angetriebene Zahnrad (6) mit einer Rolle (7) verbunden ist, die auf dem unteren Bolzen (9) sitzt.

12. Anordnung nach Anspruch 11, wobei dass auf jede der Rollen (7) zumindest teilweise ein Zugband gewickelt ist, wobei ein Ende des Zugbandes an dem weiteren Ende der Sonde befestigt ist und eine Bewegung in einer Ebene bewirkt.

13. Endoskop-Halterung, die mit einer medizinischen Sonde verbunden ist, wobei dass das in einem der Ansprüche 1 bis 12 definierte mechanische System in einem Gehäuse (11) der Halterung eingeschlossen ist und die oberen Bolzen (8) und unteren Bolzen (9) mit dem Gehäuse (11) verbunden sind.

14. Endoskop-Halterung nach Anspruch 13, wobei, dass die Anordnung zur Steuerung des weiteren Endes der Sonde in dem Gehäuse (11) so angebracht ist, dass die oberen Bolzen (8) und die unteren Bolzen (9) der Anordnung mit ihren äußeren Enden in den im Gehäuse (11) ausgebildeten Löchern platziert sind.

15. Endoskop-Halterung nach Anspruch 13, wobei vier obere Bolzen (8) in einem Winkel von 90 Grad zueinander am Ständer (2a) und mindestens zwei untere Bolzen (9) in einem Winkel von 90 Grad zueinander am Ständer (2a) befestigt sind und der Ständer (2a) mit mindestens einer Wand des Gehäuses (11) verbunden ist.

## Revendications

1. Système mécanique de commande du mouvement de la pointe distale d'une sonde médicale, comprenant un levier mobile et des poulies sur lesquelles sont enroulés les câbles tendus reliés à la pointe distale inclinable de la sonde, dans lequel le levier (1) est monté mobile sur une articulation à au moins deux degrés de liberté, qui est monté dans la base (2a),
et à la partie proximale de la base (2a) quatre tiges supérieures (8) sont fixées à un angle de 90 degrés l'une par rapport à l'autre, et à la partie distale de la base (2a) au moins deux tiges inférieures (9) sont fixées à un angle de 90 degrés l'une par rapport à l'autre, et en plus, les extrémités du guide supérieur (3) sont fixées à deux tiges supérieures opposées l'une par rapport à l'autre (8), et les extrémités du guide inférieur (4) sont fixées à deux autres tiges supérieures opposées l'une par rapport à l'autre (8), et le guide supérieur (3) est guidé de manière mobile par le levier (1) au-dessus du guide inférieur (4) guidé de manière mobile par le levier et en plus le mouvement d'un seul guide ou de deux guides est déterminé simultanément par le mouvement du levier (1), et en plus lorsque le levier (1) est déplacé parallèlement à l'axe de rotation d'un des guides et perpendiculairement à l'axe de rotation de l'autre guide, seul le second guide est déplacé
et en plus, à deux tiges supérieures (8) disposées perpendiculairement sont fixés des pignons entraîneurs (5), et chaque pignon entraîneur (5) forme un engrenage avec un pignon entraîneur correspondant (6) fixé à une tige inférieure correspondante (9), et chaque pignon entraîneur (6) est connecté à une poulie (7) fixée à la tige inférieure (9), et au moins partiellement un câble de tension est enroulé sur la poulie (7).

2. Système selon la revendication 1, dans lequel le diamètre du pignon entraîneur (5) est supérieur au diamètre du pignon entraîné (6) ou le diamètre du pignon entraîneur (5) est inférieur au diamètre du pignon entraîné (6).

3. Système selon la revendication 1, dans lequel le guide supérieur (3) et le guide inférieur (4) sont en forme de U.

4. Système selon la revendication 1, dans lequel sur chacune des poulies (7) est enroulé un seul câble tendu de sorte que les deux extrémités du câble tendu, qui sont connectées à la pointe distale de la sonde, assurent le déplacement de la pointe distale de la sonde dans un plan.

5. Système selon les revendications 1 ou 2 ou 3 ou 4, dans lequel les tiges supérieures (8) et les tiges inférieures (9) sont montées dans la base (2a) de sorte que dans la base ( 2a) sont formées des douilles dont la forme correspond à la forme des extrémités des tiges pour permettre leur montage et la rotation autour de leur propre axe.

6. Système selon les revendications 1 ou 2 ou 3 ou 4 , dans lequel les tiges supérieures (8) et les tiges inférieures (9) sont montées immobiles dans la base (2a), et chacun des guides (3,4) est directement et immobile connecté à au moins un pignon entraîneur (5), qui est monté mobile sur une tige supérieure (8).

7. Système selon l'une quelconque des revendications 1-6, dans lequel les quatre tiges supérieures (8) et les quatre tiges inférieures (9) sont montées dans la base (2a).

8. Système selon l'une quelconque des revendications 1-7, dans lequel le guide supérieur (3) est guidé mobile par le levier (1) et le guide supérieur (3) est situé au-dessus du guide inférieur (4), qui est guidé mobile par le levier (1), et les guides (3, 4) sont guidés mobiles par le levier (1) avec des trous traversants dans le levier (1), qui sont situés dans le passage supérieur (la) et le passage inférieur (1b) reliés de manière mobile.

9. Système selon l'une quelconque des revendications 1-7, dans lequel le guide supérieur (3) est guidé mobile par le levier (1) et le guide supérieur (3) est situé au-dessus du guide inférieur (4), qui est guidé mobile par le levier (1), et les guides (3, 4) sont guidés mobiles par le levier (1) de sorte que des fentes traversantes longitudinales (3a, 4a) sont formées dans le guide supérieur (3) et dans le guide inférieur (4), dans lesquels se déplace le levier (1).

10. Système selon la revendication 1, dans lequel'onutilise une rotule (2) à trois degrés de liberté ou un joint universel (14) à deux degrés de liberté.

11. Système selon la revendication 7, dans lequel le pignon entraîneur (5) est monté sur chaque tige supérieure (8), et chaque pignon entraîneur (5) forme un engrenage avec le pignon entraîneur (6), qui est monté sur chaque tige inférieur (9), et chaque pignon entraîneur (6) est connecté à la poulie (7), qui est montée sur la tige inférieure (9).

12. Système selon la revendication 11, dans lequel sur chacune des poulies (7) est au moins partiellement enroulé un câble tendu, et l'une des extrémités du câble tendu est fixée à la partie distale de la pointe et provoque un mouvement dans un plan.

13. Poignée d'endoscope connectée à la sonde médicale, dans laquelle le système mécanique défini selon l'une quelconque des revendications de 1 à 12 est fermé dans le logement (11) de la poignée, et les tiges supérieures (8) et les tiges inférieures (9) sont connectées au logement (11).

14. Poignée d'endoscope selon la revendication 13, dans laquelle le système de commande de la pointe distale de la sonde est monté dans le logement (11) de sorte que les tiges supérieures (8) et les tiges inférieures (9) du système sont installées par les extrémités externes dans les trous formés dans le logement (11).

15. Poignée d'endoscope selon la revendication 13, dans laquelleles quatre tiges supérieures (8) sont fixées à la base (2a) à un angle de 90 degrés l'une par rapport à l'autre, et au moins deux tiges inférieures (9) sont fixées à la base (2a) à un angle de 90 degrés l'une par rapport à l'autre, et la base (2a) est connectée à au moins un mur du logement (11).
